(19) Europäisches Patentamt
European Patent Office
Office européen des brevets

(11) **EP 4 520 720 A1**

# EUROPEAN PATENT APPLICATION
published in accordance with Art. 153(4) EPC

(43) Date of publication:
**12.03.2025 Bulletin 2025/11**

(21) Application number: **24767026.8**

(22) Date of filing: **29.02.2024**

(51) International Patent Classification (IPC):
*C01B 33/18* $^{(2006.01)}$    *A61Q 1/00* $^{(2006.01)}$
*A61Q 5/00* $^{(2006.01)}$    *A61Q 11/00* $^{(2006.01)}$
*A61Q 19/00* $^{(2006.01)}$    *B01J 20/283* $^{(2006.01)}$
*B01J 32/00* $^{(2006.01)}$    *B01J 35/60* $^{(2024.01)}$
*B01J 37/00* $^{(2006.01)}$    *B01J 37/08* $^{(2006.01)}$

(52) Cooperative Patent Classification (CPC):
A61Q 1/00; A61Q 5/00; A61Q 11/00; A61Q 19/00;
B01J 20/283; B01J 21/00; B01J 35/60;
B01J 37/00; B01J 37/08; C01B 33/18

(86) International application number:
**PCT/JP2024/007618**

(87) International publication number:
**WO 2024/185656 (12.09.2024 Gazette 2024/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **06.03.2023 JP 2023033927**

(71) Applicant: **TOKUYAMA CORPORATION
Yamaguchi 745-8648 (JP)**

(72) Inventor: **YOSHIMURA, Noriko
Shunan-shi, Yamaguchi 745-8648 (JP)**

(74) Representative: **Viering, Jentschura & Partner
mbB
Patent- und Rechtsanwälte
Am Brauhaus 8
01099 Dresden (DE)**

(54) **POROUS SPHERICAL SILICA, CATALYST CARRIER, COSMETIC, ANALYSIS COLUMN, POLISHING AGENT, RESIN COMPOSITION, AND METHOD FOR PRODUCING POROUS SPHERICAL SILICA**

(57) Provided are an independent porous spherical silica such that the alkali metal content is reduced, the hardness of the particles is increased, and the number of shred particles is reduced; and a method of producing such a porous spherical silica. In the porous spherical silica, the 50% cumulative diameter (D50) of volume based particle size distribution is 2 $\mu$m to 200 $\mu$m, the ratio (D10/D90) of the 10% cumulative diameter (D10) thereof to the 90% cumulative diameter (D90) thereof is at least 0.3, the pore volume is 0.5 mL/g to 8 mL/g, the mode pore radius is 5 nm to 50 nm, the specific surface area is 50 m$^2$/g to 400 m$^2$/g, and the arithmetic mean value of "compressive test forces when specimens are found to break" obtained according to a method specified in JIS Z8844 is $1.0 \times 10^1$ mN to $2.0 \times 10^1$ mN, the number of particles each having a circularity of at most 0.7 is at most 20, and the alkali metal content is at most 50 ppm.

**EP 4 520 720 A1**

## Description

[Technical Field]

The present invention relates to a novel porous spherical silica, catalyst carrier, cosmetic, analytical column, polish, resin composition, and method of producing a porous spherical silica.

[Background Art]

Porous silica has been researched in a variety of ways, and porous silicas having a variety of physical properties have been proposed. Examples of the method of producing porous silica includes methods of: neutralizing an alkali metal silicate aqueous solution by adding a mineral acid to the solution, and separating and collecting generated particles (patent literature 1); spray-drying a fumed silica-dispersed solution (patent literature 2); and gelating a fumed silica-dispersed solution in a solution (patent literature 3). Porous spherical silica produced by any of the foregoing methods has a unique pore volume and pore diameter, and is expected to be used as carriers for catalysts, and fillers for analytical columns. Further, when used as polishes for industrial products and the like, the porous spherical silica is fixed to resin on a polishing pad easily by permeation of the resin into the insides of the pores. Using spherical products for the foregoing cases leads to easy adjustment of the particle sizes, and easy improvement in filling-rate, which are advantages. In addition, porous spherical silica gives a smooth touch derived from the shape thereof, and thus, is also useful as additives for cosmetics.

[Citation List]

[Patent Literature]

[Patent Literature 1] WO 2004/101139 A1
[Patent Literature 2] WO 2019/131873 A1
[Patent Literature 3] WO 2022/154014 A1

[Non Patent Literature]

[Non Patent Literature 1] Sintering of fine ceramics. Journal of the Society of Powder Technology. 1988, vol. 25, no. 12, pp. 805-811

[Summary of Invention]

[Technical Problem]

However, since being fragile under a pressure derived from a porous structure thereof, porous silica has a problem in durability when used for carriers for catalysts which are used in a long term, and when used for fillers for analytical columns which are used in fluid. In addition, when porous silica is used for polishes for industrial products and the like, the shapes of the particles warp and/or the particles break in the step of kneading with resin to deteriorate handleability, which is problematic. In general, a firing method is used (non patent literature 1) as the method of increasing the hardness of an inorganic material, whereas the porous silica disclosed in patent literature 1, which uses sodium silicate as a raw material, cannot maintain the porous structure thereof because drop in melting point caused by the sodium content coming from the raw material leads to blockage to the pores in firing at a high temperature.

The porous silica disclosed in patent literature 2, which is obtained by spray-drying a fumed silica-dispersed solution, has a low compressive strength after firing. Thus, a silica having an increased hardness cannot be obtained. In the porous silica disclosed in patent literature 3, which is obtained by gelating a fumed silica-dispersed solution in the solution, the circularity as the entire particles deteriorates because a shred fine powder is generated in a disintegrating step necessary due to agglomeration of the particles by firing. This inclusion of fine powder causes blockage to analytical columns when the porous spherical silica is used for fillers for the columns, and deteriorates the touch of additives for cosmetics when the porous spherical silica is used as the additives.

Therefore, an object of the present invention is to provide: an independent porous spherical silica such that the alkali metal content is reduced, the hardness of the particles is increased, and the number of shred particles is reduced; and a method of producing such a porous spherical silica.

[Solution to Problem]

As a result of their intensive research for solving the aforementioned problems, the inventor of the present invention etc. found out that an independent porous spherical silica such that the alkali metal content is reduced, the hardness of the particles is increased, and the number of shred particles is reduced can be produced by, in the process of producing a porous spherical silica, spray-drying and thereafter firing gels obtained by gelating a fumed silica-dispersed solution in the solution, and reached the completion of the present invention.

[1] A porous spherical silica, wherein

a 50% cumulative diameter (D50) of volume based particle size distribution measured by a Coulter counter method is in a range of 2 μm and 200 μm,
a ratio (D10/D90) of a 10% cumulative diameter (D10) of the distribution to a 90% cumulative diameter (D90) of the distribution is at least 0.3,
a pore volume by a BJH method is 0.5 mL/g to 8 mL/g,
a mode pore radius by the BJH method is 5 nm to 50 nm,
a specific surface area by a BET method is 50 $m^2$/g to 400 $m^2$/g, and
an arithmetic mean value of "compressive test forces when specimens are found to break" is $1.0 \times 10^1$ mN to $2.0 \times 10^1$ mN, the specimens being ten particles, the compressive test forces being obtained according to a method specified in JIS Z8844 when a loading speed is 0.4462 mN/sec,
a number of particles each having a circularity of at most 0.7 is at most 20, the particles being among 200 particles observed in an SEM image, and
an alkali metal content is at most 50 ppm.

[2] A catalyst carrier comprising the porous spherical silica according to [1].
[3] A cosmetic comprising the porous spherical silica according to [1].
[4] An analytical column comprising the porous spherical silica according to [1].
[5] A polish comprising the porous spherical silica according to [1].
[6] A resin composition comprising the porous spherical silica according to [1].
[7] A method of producing a porous spherical silica, the method comprising:

preparing a W/O emulsion comprising an aqueous phase where a fumed silica is dispersed, and an organic phase including a nonaqueous solvent as a major component;
heating the emulsion and gelating the aqueous phase to obtain a gel-dispersed solution;
collecting, from the solution, and spray-drying generated gels; and
firing obtained porous spherical silica at a temperature of 900 to 1500°C for 10-25 hours.

[8] The method of producing a porous spherical silica according to [7], wherein
a spraying method in the spray-drying was a method using a two-fluid nozzle.

[Advantageous Effects of Invention]

The porous spherical silica according to the present invention has a large pore volume, and, as indicated by the strength of the "compressive test forces when specimens are found to break", an increased hardness. Therefore, the porous spherical silica can maintain the porous structure thereof without breaking of the particles or blockage to the pores even when used in a long term or in fluid. Thus, using the porous spherical silica according to the present invention can lead to improvement in durability, which is demanded for carriers for catalysts, adsorbents for carbon dioxide etc., and fillers for analysis columns. Further, when the porous spherical silica according to the present invention is used as polishes for industrial products and the like, the particles are so strong that kneading with resin can be withstood, and the handleability in the production process is improved.

In addition, because of a narrow particle size distribution as shown by the high D10/D90, blockage due to fine powder entering the spaces among the particles is less likely when the porous spherical silica according to the present invention is used as fillers for analytical columns.

Further, the number of particles each having a circularity of at most 0.7 is at most 20 among 200 particles observed in a SEM image; that is, the number of shred particles is reduced. Because the particles are in the form of independent sphere, using the porous spherical silica according to the present invention as additives for cosmetics can give a smooth touch.

In addition, the alkali metal content is at most 50 ppm; that is, the purity is high, which is extremely useful especially as a polish intended for materials that dislike containing alkali metals, such as semiconductors.

In the production method according to the present invention, agglomeration of the particles is suppressed by spray-drying, and thereafter firing the base material, which enables production by a process including no disintegrating step. This can suppress shred particles that can be generated when a disintegrating step is performed, and allows a porous spherical silica having the aforementioned features to be produced without generating fine powder, or deteriorating the circularity as the entire particles.

In addition, in the production method according to the present invention, the strength of the aggregation of the primary particles is improved by firing under specific conditions. This makes it possible to obtain a porous spherical silica such that the hardness of the particles is increased .

[Description of Embodiments]

The following embodiments are examples of the present invention. The present invention is not limited to these embodiments.

<Porous Spherical Silica>

The porous spherical silica according to the present invention is such that: the 50% cumulative diameter (D50) of the volume based particle size distribution measured by a Coulter counter method is in the range of 2 $\mu$m and 200 $\mu$m; and the ratio of the 10% cumulative diameter (D10) of this distribution to the 90% cumulative diameter (D90) of this distribution (D10/D90) is at least 0.3. The porous spherical silica in the foregoing ranges is less likely to cause blockage to analytical columns, and makes filling easy when used as fillers for the columns. The D50 is preferably 2 $\mu$m to 100 $\mu$m, particularly preferably 5 $\mu$m to 50 $\mu$m, and further preferably 5 $\mu$m to 20 $\mu$m. The D10/D90 is preferably at least 0.4, and further preferably at least 0.5. The D10/D90 cannot exceed 1.0, and generally, is at most 0.6.

The porous spherical silica according to the present invention has a pore volume of 0.5 mL/g to 8 mL/g: the pore volume is measured by the following BJH method. It is difficult to obtain a porous spherical silica having a large pore volume exceeding 8 mL/g. To produce a porous spherical silica having a pore volume of at most 6 mL/g is easier, to produce a porous spherical silica having a pore volume of at most 4 mL/g is much easier, and to produce a porous spherical silica having a pore volume of at most 2.5 mL/g is particularly easier. In particular, in order for the porous spherical silica according to the present invention to have a large carried volume or adsorption volume when this porous spherical silica is used as carriers for catalysts, or adsorbents for carbon dioxide etc., the pore volume thereof is preferably at least 0.6 mL/g, further preferably at least 0.7 mL/g, and more preferably at least 1.0 mL/g.

The mode pore radius by the BJH method is at least 5 nm, preferably at least 10 nm, and more preferably at least 15 nm. The upper limit is at most 50 nm, and preferably at most 30 nm.

The pore volume and pore radius by the BJH method are obtained by drying a sample to be measured in a vacuum of at most 1 kPa at 200°C for at least 3 hours, and thereafter, obtaining and analyzing an adsorption isotherm in the adsorption of nitrogen only at the liquid-nitrogen temperature by the BJH method (Barrett, E. P., Joyner, L. G., Halenda, P. P. J. Am. Chem. Soc. 1951, 73, 373). "The mode pore radius by the BJH method" means the value of the pore radius when a pore distribution curve (volume distribution curve) takes the maximum value: the pore distribution curve is plotted as the differential of the cumulative pore volume by the logarithm of the pore radius obtained by the analysis by the BJH method is on the vertical axis, and the pore radius is on the horizontal axis.

The porous spherical silica according to the present invention has a specific surface area by the BET method of 50 $m^2$/g to 400 $m^2$/g. This specific surface area is preferably at least 100 $m^2$/g, and particularly preferably 120 $m^2$/g to 350 $m^2$/g. The larger the specific surface area of a fumed silica used as a raw material is, the larger the specific surface area of the porous spherical silica is. In addition, the larger the specific surface area of the fumed silica is, the easier turning into gels is, and the easier formation into a sphere is. However, because the specific surface area of fumed silica is generally at most 400 $m^2$/g, it is difficult to obtain a porous spherical silica having a specific surface area exceeding 400 $m^2$/g. It is noted that the specific surface area is a value by a BET multipoint method using nitrogen adsorption.

The arithmetic mean value of "compressive test forces when specimens are found to break" (hereinafter "breaking compressive test force") is $1.0 \times 10^1$ to $2.0 \times 10^1$ mN where the specimens are ten particles of the porous spherical silica according to the present invention, and the compressive test forces are obtained according to a method specified in JIS Z8844:2019 when the loading speed is set in 0.4462 mN/sec. When the breaking compressive test force is more than $2.0 \times 10^1$ mN, it is difficult to obtain a porous spherical silica having a pore volume of at least 0.5 mL/g. The breaking compressive test force is preferably $1.0 \times 10^1$ to $1.8 \times 10^1$ mN. The breaking compressive test force within any of the above ranges leads to a porous spherical silica such that the hardness of the particles thereof is increased, that has target durability.

The porous spherical silica according to the present invention has an alkali metal content of at most 50 ppm (on the mass basis). In particular, the alkali metal content is preferably at most 40 ppm, and more preferably at most 30 ppm.

The porous spherical silica according to the present invention has a spherical shape. Here, the spherical shape means that the average circularity obtained by image analysis using a Scanning Electron Microscope (SEM) is at least 0.8. "Average circularity obtained by image analysis" is the arithmetic mean value of the circularities obtained by subjecting, to image analysis, a SEM image of at least 2000 particles of the porous spherical silica which is observed with a SEM at the magnification of 1000. Here, "circularity" is the value calculated by the following equation (1):

$$(1): C = 4\pi S / L^2$$

In the equation (1), C means the circularity, S means the area of the porous spherical silica in the image (project area), and L means the length of the circumference (perimeter) of the porous spherical silica in the image. The average circularity is particularly preferably at least 0.85.

In the porous spherical silica according to the present invention, the number of shred particles among 200 particles observed in a SEM image is at most 20. Here, the shred particle means particles each having a circularity of at most 0.7: the circularity is calculated according to the aforementioned definition. The number of the shred particles is preferably at most 15, and more preferably at most 10.

The porous spherical silica according to the present invention may be hydrophilic and may be hydrophobic. The porous spherical silica according to the present invention that is produced by the undermentioned production method is hydrophilic. A hydrophobic porous spherical silica can be obtained by, for example, obtaining the hydrophilic porous spherical silica by the undermentioned production method, and thereafter, appropriately using a silica surface treating method. "Hydrophilic" here means being dispersible in water containing no organic solvent.

The porous spherical silica according to the present invention has the foregoing features, and thus, can be used as carriers for catalysts or perfumes, adsorbents for carbon dioxide etc., fillers for analytical columns, additives for cosmetics, polishes for industrial products, or additives for various resin compositions.

<Method of Producing Porous Spherical Silica>

The method of producing the porous spherical silica according to the present invention is not particularly limited. The aforementioned large pore volume and large modal diameter of the pore radius are easily realized by the use of a fumed silica-dispersed solution as a raw material. In general, fumed silica has the structure of aggregated fine particulate silica (primary particles). Therefore, using a fumed silica-dispersed solution as a raw material of the porous spherical silica, and turning fumed silica into gels in the dispersed solution to form a network leads to a suppressed decrease in pore volume due to drying shrinkage, and makes it possible to obtain a porous spherical silica having a large pore volume.

A more specific example is the method of producing the porous spherical silica including: preparing a W/O emulsion including an aqueous phase where a fumed silica is dispersed, and an organic phase including a nonaqueous solvent as the major component (step of preparing a W/O emulsion); next, heating the emulsion and gelating the aqueous phase to obtain a porous spherical silica-dispersed solution (gelating step); and thereafter, collecting, from the solution (step of collecting gels), spray-drying (drying step), and firing (firing step) the generated porous spherical silica. Hereinafter each of the steps will be described in detail.

(Step of Preparing W/O Emulsion)

As a method of preparing a W/O emulsion including an aqueous phase where a fumed silica is dispersed, and an organic phase including a nonaqueous solvent as the major component, the following method is particularly preferably used: first, a dispersed solution obtained by dispersing a fumed silica in an aqueous phase is prepared (step of preparing a dispersed solution), and by using this solution and an organic solvent, an emulsion is prepared according to a usual way (step of forming an emulsion). These steps will be further described below.

(Step of Preparing Dispersed Solution)

The step of preparing a dispersed solution is the step of dispersing a fumed silica in water to prepare a dispersed solution.

A fumed silica as used herein is dispersible in water, and can turn into gels by heating, adjusting pH, etc. Such properties can be realized when there are a large number of silanol groups on the surface of silica. Thus, most of fumed silicas can be used unless the fumed silicas are subjected to what is called surface treatment. In view of easy progress in gelating, a fumed silica having a specific surface area of at least 100 $m^2/g$, particularly at least 200 $m^2/g$ is preferably used. This specific surface area is further preferably at least 250 $m^2/g$. The larger the specific surface area is, the faster the speed of progress in gelating is, and the easier it is to gelate droplets (W-phase) where the fumed silica is dispersed. In view of availability, the upper limit of the specific surface area of a fumed silica to be used is preferably 400 m$^2$/g. It is noted that the specific surface area is a value by a BET multipoint method using nitrogen adsorption.

The larger the specific surface area of a fumed silica to be used as a raw material is, the larger the specific surface area of the porous spherical silica obtained by the method described here is. Thus, appropriately selecting a fumed silica to be used as a raw material according to the specific surface area of the target porous spherical silica enables the specific surface area of the porous spherical silica to be controlled freely without any change in producing conditions. Fumed silicas having different specific surface areas can be used in combination in the present invention.

Fumed silicas as described above are commercially available. For example, the following is available: various hydrophilic grades of REOLOSII, from Tokuyama Corporation, various hydrophilic grades of AEROSIL from NIPPON AEROSIL CO., LTD., and various hydrophilic grades of dry silica HDK from Wacker Asahikasei Silicone Co., Ltd.

In general, fumed silica is of high purity, and contains almost no impurities such as alkali metals. Thus, the alkali metal content of the produced porous spherical silica can be extremely low.

Water is essential as a solvent in this step, whereas any other solvent may be contained as long as not hindering the formation of the emulsion, and the subsequent gelating. When a latent base is used for promoting the undermentioned gelating, it is preferable to dissolve the latent base in water before the fumed silica is dispersed.

As a method of dispersing the fumed silica in the solvent, preferably, a dispersed solution where the fumed silica is predispersed in the solvent is prepared, and is subjected to fine dispersion with a disintegrator or the like. Specific examples of a disintegrator that can be used for the fine dispersion include a ball mill, a bead mill, a vibration mill, a pin mill, an atomizer, a colloid mill, a homogenizer, a high pressure homogenizer, and an ultrasonic homogenizer. The extent of the dispersion after the fine dispersion is preferably such that the value of the D90 is at most 0.5 $\mu$m when the particle size distribution of the dispersed solution is measured by a laser diffraction scattering method.

The silica concentration of the fumed silica-dispersed solution is preferably in the range of 10 mass% and 30 mass%, is more preferably at least 15 mass%, and is particularly preferably at least 20 mass%. The higher the silica concentration of the fumed silica-dispersed solution is, the faster the speed of progress in gelating is. Too high a silica concentration however leads to loss of fluidity, which makes it difficult to form a fumed silica-dispersed solution.

The gelation of the fumed silica-dispersed solution is accelerated by heating. Progress in gelation of the fumed silica-dispersed solution at the stage of the step of preparing a dispersed solution causes the W-phase to be difficult to form spheres in the subsequent step of forming an emulsion, and in an extreme case, makes it difficult to form the emulsion itself. Therefore, in the step of preparing a dispersed solution, the temperature of the fumed silica-dispersed solution is preferably kept at approximately room temperature (20°C) or lower. It is also effective to cool the fumed silica-dispersed solution to a temperature lower than room temperature (preferably at most 15°C, more preferably at most 12°C) when the specific surface area of the fumed silica is large and/or the concentration thereof is high, and the gelating easily progresses.

(Step of Forming Emulsion)

The step of preparing a W/O emulsion is the step of dispersing, in a nonaqueous solvent, the fumed silica-dispersed solution obtained in the step of preparing a dispersed solution, and forming the W/O emulsion. When such a W/O emulsion is formed, the fumed silica-dispersed solution, which is a dispersoid, is in the form of sphere because of surface tension etc. Thus, spherical gels can be obtained by gelating the fumed silica-dispersed solution in the form of sphere that is dispersed in the nonaqueous solvent.

Any nonaqueous solvent is used in this production method as long as being so hydrophobic that the solvent and the fumed silica-dispersed solution can form an emulsion. As such a solvent, for example, an organic solvent of any of hydrocarbons, halogenated hydrocarbons, and the like can be used. More specific examples of such a solvent include nonaqueous solvents such as hexane, heptane, octane, nonane, decane, liquid paraffin, dichloromethane, chloroform, carbon tetrachloride, and dichloropropane. Among them, hexane, heptane, and decane, which have a moderate viscosity, can be preferably used. If necessary, a plurality of solvents may be used in combination. A hydrophilic solvent such as lower alcohols can be also used in combination (used as a mixed solvent) as long as such a hydrophilic solvent and the fumed silica-dispersed solution can form an emulsion.

The amount of using the nonaqueous solvent is not particularly limited as long as the W/O emulsion can be formed. Generally, approximately 1 to 10 parts by volume of the nonaqueous solvent is used per 1 part by volume of the fumed silica-dispersed solution.

In this production method, a surfactant is preferably added when the W/O emulsion is formed. As a surfactant as used herein, any of known surfactants that are used for forming W/O emulsions can be used without limitations, and any of anionic surfactants, cationic surfactants, and nonionic surfactants can be used. Among them, nonionic surfactants are preferrable because of easy formation of the W/O emulsion, and because alkali metals are hardly contaminated. In particular, a surfactant having a HLB value of 3 to 5 can be preferably used: the HLB value indicates the degree of hydrophilicity or hydrophobicity of a surfactant, and here, means the HLB value according to Griffin's method. Specific examples of surfactants that can be preferably used here include sorbitan monooleate, sorbitan monostearate, and sorbitan monosesquioleate.

The amount of using the surfactant is the same as a usual amount thereof when a W/O emulsion is formed. Specifically, any amount in the range of 0.05 g and 10 g can be preferably applied for 100 mL of the fumed silica-dispersed solution.

As the method of dispersing the fumed silica-dispersed solution in the nonaqueous solvent when the W/O emulsion is formed, a known method of forming a W/O emulsion can be used. In view of easy industrial production etc., emulsion formation by mechanical emulsification is preferable, and specific examples thereof include methods using a mixer, a homogenizer, and the like. Preferably, a homogenizer can be used. Through this step of forming an emulsion, an emulsion having a sharp particle size distribution of the droplets of the aqueous phase is obtained. Thus, the particle size distribution of the finally obtained spherical porous silica is also sharp. When particles of preferably at least 1 $\mu$m and less than 100 $\mu$m, more preferably 8 $\mu$m to 90 $\mu$m are obtained, the rotation speed of the homogenizer is preferably 1,000 rpm to 15,000 rpm, more preferably 2,000 rpm to 12,000 rpm, and further preferably 4,000 rpm to 9,000 rpm. The stirring time is preferably 30 seconds to 1 hour, and more preferably 1 minute to 30 minutes.

(Gelating Step)

The gelating step follows the step of preparing a W/O emulsion, and is the step of gelating the fumed silica-dispersed solution in the state where the droplets of the fumed silica-dispersed solution are dispersed in the nonaqueous solvent. This gelating can be carried out by a known method. For example, the gelating can be easily progressed by the technique of heating to a high temperature, or the technique of adjusting the pH of the fumed silica-dispersed solution to be weakly acidic, or basic. These techniques are preferable because the reaction thereby can be actively controlled. It is noted that the pH of the fumed silica-dispersed solution prepared by the aforementioned method without pH adjustment is generally in the range of 3.0 and 4.5.

The heating should be performed so that the temperature does not exceed the boiling point of each of the used solvents. The lower limit of the gelating temperature is preferably 50°C, and more preferably 60°C; and the upper limit thereof is preferably at most 100°C, and more preferably at most 90°C.

The aforementioned pH adjustment can be easily performed by: the method of mixing a substance that thermally decomposes by heating so as to show basicity (which will be referred to as a "latent base"), such as urea, with the fumed silica-dispersed solution in advance, and heating the mixture in the gelating to raise the pH; or the method of adding a base into the W/O emulsion while maintaining the W/O emulsion forming state by stirring with a mixer or the like.

Specific examples of this base include: ammonia; tetraalkylammonium hydroxides such as tetramethylammonium hydroxide (TMAH); amines such as trimethylamine; alkali hydroxides such as sodium hydroxide; alkali metal carbonates such as sodium carbonate, and sodium hydrogen carbonate; and alkali metal silicates. The strength of the aforementioned stirring may be such that the W/O emulsion and the base are mixed.

Among the aforementioned pH adjusting methods, the method by thermal decomposition of a latent base such as urea, or the method using ammonia, a tetraalkylammonium hydroxide, or an amine as a base is preferable in view of no inclusion of metal elements. When used for the pH adjustment, ammonia may be blown in as gas, or may be added as aqueous ammonia. It is particularly preferable to apply pH adjustment using urea because the pH can be uniformly adjusted as a whole by heating.

As a pH when the pH is adjusted to promote the gelating, it is particularly preferable to adjust the amount of the addition so that the value of the pH of the fumed silica-dispersed solution rises to approximately 4.5 to 8.0. This is also true of the case where a latent base is used. For example, when urea is used, the following are specific amounts of the addition on the basis of the fumed silica-dispersed solution: preferably at least 1 mass%, and particularly preferably at least 2 mass%; and as the upper limit, preferably at most 7 mass%, and more preferably at most 5 mass%.

In order to prevent the gels from agglomerating, stirring is preferable when the aforementioned heating and pH adjustment are performed. A known method is generally used for the stirring. For example, specifically, a mixer with stirring blades can be used.

After the gelating, since the dispersoid have changed from the form of liquid to the form of solid, the system is not a W/O emulsion but a dispersed solution (suspension) where solids (gels) are dispersed in the hydrophobic solvent.

(Step of Collecting Gels)

In this production method, the gels generated as described above are collected from the solution. As a method of collecting the gels, a general solid-liquid separating method such as filtration and centrifugation can be used. Prior to the collection, WO phase separation may be performed. WO phase separation is to separate the gel-dispersed solution into two layers of an O-phase and a W-phase, and is the operation generally called demulsification. Here, the gels obtained in the gelating step is present on the separated W-phase side. Separating this from the O-phase makes it easy to collect the gels through solid-liquid separation by filtration or the like.

As the WO phase separation method, one may appropriately select and carry out a known method as a demulsifying method. Preferably, the WO phase separation is performed by adding a certain amount of an aqueous organic solvent that is commonly used in demulsification to the gel-dispersed solution, and heating the resultant to separate the resultant into the O-phase and the W-phase. After this step, generally, the upper layer is the O-phase (layer mainly including the organic solvent), and the lower layer is the W-phase (water layer including the aqueous organic solvent and the gels).

Examples of the aqueous organic solvent include acetone, methanol, ethanol, and isopropyl alcohol. Among them, isopropyl alcohol can be particularly preferably used.

Preferably, the amount of the addition of the aqueous organic solvent is adjusted according to the type and the amount of the surfactant used when the emulsion was formed, which has a HLB of 3 to 5. For example, when sorbitan monooleate is used as the surfactant, the demulsification can be preferably performed by adding the aqueous organic solvent approximately 1/6 to 1/2 times as much as the non-aqueous organic solvent (aqueous organic solvent/non-aqueous organic solvent) by mass, stirring the resultant if necessary, and thereafter, allowing the resultant to stand.

In the WO phase separation, the surfactant moves to the O-phase side (extraction). Thus, a porous spherical silica with which no impurities from the surfactant are mixed can be obtained by removing the O-phase.

The temperature range for the heating is at least 50°C, preferably approximately 50 to 80°C, and more preferably approximately 60 to 70°C.

After the aqueous organic solvent is added to the gel-dispersed solution as described above, it is preferable to stir the resultant in order to prevent the gels from agglomerating with each other. Generally, a known method is used for the stirring. Specifically, for example, a mixer with stirring blades can be used. The extent of the mixing is not particularly limited, but may be such that there is rotation caused by the stirring in the level of the dispersion, which is, for example, in the case of the stirring with a mixer, 0.1 to 3.0 kW/m$^3$, preferably 0.5 to 1.5 kW/m$^3$. As the stirring time, 0.5 to 24 hours, preferably approximately 0.5 to 1 hour are proper.

After the WO phase separation, the W-phase including the gels are collected. Specifically, the O-phase (upper layer) can be separated and removed by, for example, decantation.

(Drying Step)

Preferably, the gels included in the collected W-phase are collected from the W-phase. A known method can be used as the collecting method. Specific examples of such a method include suction filtration, and centrifugation. At this time, preferably, the solvent is removed, so that the collected gels are in the form of cake.

The collected gels are dried by spray-drying. When gels are produced by a wet process as in the production method according to the present invention, capillary force of the solvent is generated among the particles of the gels when the gels are taken out from the solution and dried. This leads to agglomeration of the particles. Therefore, particularly in stationary drying, dry powder also agglomerates. On the other hand, the drying by spray-drying makes it possible for the diameters of the droplets of the solvent in the drying to approach the particle sizes of the porous spherical silica particles. Ideally, the condition when the diameters of the droplets of the solvent are equal to the particle sizes of the porous spherical silica particles is that the porous spherical silica particles are contained in the droplets of the solvent in the drying, respectively. In the above condition, because the porous spherical silica particles are not adjacent to each other in the drying, the drying can be carried out in a state where no capillary force caused by the solvent is generated among the particles, and it is easy to obtain particles without agglomeration. The spray-drying method is not particularly limited, but a known method such as a rotary atomizer method and a nozzle method can be used. Such a method is preferably selected so that the target particle sizes of the porous spherical silica particles, and the diameters of the droplets of the solvent to be sprayed are equal. Generally, the diameter of a sprayed droplet tends to be finer by a nozzle method than by a rotary atomizer method. In view of capability of obtaining finer diameters of the droplets, and difficulty in agglomeration in the drying, a method using a two-fluid nozzle is particularly preferrable.

Preferably, the gels in the spray-drying are dispersed in an aqueous organic solvent in view of difficult agglomeration. Specific examples of an aqueous organic solvent herein include acetone, methanol, ethanol, and isopropyl alcohol. Using a solvent having a lower boiling point can improve drying efficiency.

Water or a nonaqueous solvent may be mixed in the above aqueous organic solvent. In order to suppress agglomeration in the drying, the concentration of the aqueous organic solvent in the solvent is preferably at least 60 mass%, more preferably at least 65 mass%, and particularly preferably at least 70 mass%. When the concentration of the aqueous organic solvent is within any of the above ranges, agglomeration due to drying failure in the spray-drying is difficult to take place. It is noted that drying shrinkage inside the pores can be adjusted according to the concentration of the aqueous solvent in the drying. Thus, adjusting the concentration of the aqueous organic solvent can lead to moderate drying shrinkage, and a controlled pore volume. Specifically, drying shrinkage is more easily caused, and the pore volume becomes smaller by raising the ratio of water contained in the solvent, and lowering the concentration of the aqueous organic solvent. On the contrary, lowering the ratio of water contained in the solvent, and raising the concentration of the aqueous organic solvent lead to suppression in drying shrinkage, and a larger pore volume.

Preferably, the aqueous organic solvent where the gels are dispersed (slurry) is stirred in order to suppress agglomeration of the particles in the solution. A generally known method can be used for the stirring method. Specific examples include a stirrer, and a mixer with stirring blades. The extent of the mixing may be such that the particles do not sediment in the solution, and the particles are not broken. The slurry may be put into a spray dryer while stirred, or may be put into a spray dryer after stirred for a certain period of time. In view of preventing sedimentation of the particles, the slurry is more preferably put while stirred.

The inlet temperature in the spray-drying is preferably adjusted so as to be at least the boiling point of the solvent of the slurry, and so that the porous spherical silica is not in the form of slurry or cake but is dry powder at the outlet of the spray dryer. "At least the boiling point" described above means at least the boiling point of the solvent at a pressure in the drying. The inlet temperature is more preferably adjusted so as to take a value higher than the boiling point of the solvent of the slurry by approximately 20 to 50°C, and is preferable adjusted so that the temperature difference between the inlet temperature and the outlet temperature is in the range of 10 and 70°C. Generally, a heat source is set only at an input port (inlet) of the spray dryer, and the outlet temperature does not exceed the inlet temperature. The temperature difference between the inlet temperature and the outlet temperature is more preferably in the range of 20 and 70°C, and particularly preferably in the range of 20 and 60°C. When the inlet temperature and the outlet temperature are within any of the above ranges, a dry state in the spray-drying becomes good, and a porous spherical silica without agglomeration is obtained.

It is sufficient that the circulating gas volume in the spray-drying is adjusted so that the temperature in a drying chamber is stabilized. Preferably, the circulating gas volume is adjusted according to the scale of the spray dryer. When the dried porous spherical silica is collected using a cyclone, the circulating gas volume affects the collection efficiency with the cyclone. The larger the circulating gas volume is, the higher the collection efficiency with the cyclone is.

It is sufficient that the concentration of the gels contained in the slurry is adjusted to such an extent that the slurry where the gels are dispersed has fluidity, and is not blocked into the spray dryer. Specifically, the concentration of the solid content of the silica after the drying (in terms of mass) is preferably at most 60 mass%, more preferably at most 50 mass%, and further preferably at most 40 mass%. The lower limit of the concentration of the gels is not particularly limited, but is preferably not extremely low because the lower the concentration is, the worse the drying efficiency is. This lower limit is more preferably at least 5 mass%.

It is sufficient that the feed rate of the slurry in the spray-drying is adjusted so that the spraying is stabilized, and the inlet temperature and the outlet temperature take any of the aforementioned values. This rate is preferably adjusted according to the scale of the spray dryer. The feed rate of the slurry affects the temperature difference between the inlet temperature and the outlet temperature. The faster the feed rate of the slurry is, the larger the temperature difference between the inlet temperature and the outlet temperature is.

As the method of collecting the dried porous spherical silica, generally, a known method can be used. Specific examples of such a method include a one-point collection method, and a two-point collection method. A general collector can be used, and specific examples thereof include a cyclone, and a bag filter. In view of removal of coarse particles, the collection is preferably performed by a two-point collection method using a cyclone.

(Firing Step)

Further, the porous spherical silica according to the present invention is fired after the drying. The breaking compressive test force can be increased by this firing. The firing conditions may be adjusted so that the breaking compressive test force takes the target value. The longer the firing time is and the higher the firing temperature is, the stronger the breaking compressive test force is. The firing temperature is 900 to 1500°C, preferably at most 1400°C, and more preferably at most 1300°C. The firing time is 10 to 25 hours, preferably 12 to 23 hours, and more preferably 12 to 20 hours. When the firing temperature and the firing time are in any of the above ranges, a porous spherical silica having a breaking compressive test force in the target range is obtained without the particles, which were dried by spray-drying so as not to agglomerate, agglomerating again, or without the pores being blocked.

A known method can be used as the firing method. Generally, an example of such a method is the method of placing, in a crucible, a quartz vat, or the like, and heating a dried porous spherical silica with an electric furnace.

The atmosphere in the firing is not particularly limited. The firing can be performed in an inert gas such as argon and nitrogen, or in an air atmosphere.

The heating rate in the firing may be in a range such that the temperature rise in a heating device such as an electric furnace can follow the heating rate. The slower the heating rate is, the worse the processing efficiency in the firing is. Thus, the heating rate is preferably not extremely slow. When a general electric furnace is used, the temperature is suitably raised at 2 to 10°C/min.

In the foregoing production method, the particle size of the obtained porous spherical silica is substantially the same as the diameter of each of the droplets (W-phase) of the fumed silica-dispersed solution in the W/O emulsion prepared in the step of forming an emulsion. Therefore, it is necessary to set the dispersion conditions so as to obtain the diameter in the target range. Various methods of controlling the diameters of droplets in a W/O emulsion are known, and any technique thereof may be appropriately selected and applied. A known method can be used for the method of adjusting the particle sizes of the droplets. Specific examples of such a method include the method of adjusting the amount of the addition of the surfactant, and the method of adjusting the shearing force applied in the emulsification by the rotation speed, the flow rate, etc. In the adjustment of the amount of the addition of the surfactant, the larger the amount of the used surfactant is, the finer the droplets tend to be; and the smaller the amount thereof is, the larger the droplets tend to be. In the adjustment using the shearing force, the stronger the applied shearing force is, the finer the droplets tend to be; and the weaker the applied shearing force is, the larger the droplets tend to be.

The pore volume can be controlled by drying shrinkage. A known method can be used as a method of controlling drying shrinkage. A specific example of such a method is the method of adjusting the concentration of the aqueous solvent before the drying. The pore volume can also be controlled by the firing conditions. The higher the firing temperature is and the longer the firing time is, the smaller the pore volume tends to be. Using fumed silica as a raw material as in the production method according to the present invention leads to the large mode pore radius because fumed silica has an aggregating structure. The specific surface area can be adjusted by appropriately selecting the specific surface area of the fumed silica used as a raw material, and can be also adjusted by the gelating time. It is noted that the shorter the gelating time is, the larger the specific surface area tends to be. The specific surface area can also be adjusted by the firing conditions. **In** general, the higher the firing temperature is and the longer the firing time is, the smaller the specific surface area tends to be.

The alkali metal content can be easily lowered if a person skilled in the art takes enough care to avoid contamination (inclusion of impurities) to perform the production using a fumed silica containing substantially no alkali metal as a raw material as described above, and other raw materials containing substantially no alkali metal. When further reduction of the alkali metal content is aimed, the cake may be washed with water, an organic solvent, or the like after the solid-liquid separation before the drying.

The number of the shred particles can be reduced by suppressing agglomeration of the particles that is caused in the production process and performing no disintegrating step as in the production method according to the present invention. Care must be taken not to give an excessive load to the particles so as not to break the particles throughout the production process.

[Examples]

Hereinafter examples for specifically describing the present invention will be shown. The present invention is not limited to these examples only.

<Evaluation Methods>

Evaluation concerning the following items was performed for every produced porous spherical silica.

(Measuring Particle Size Distribution and Volume-Based Cumulative Diameter with Coulter Counter)

To 40 mL of an ion-exchanged water, 0.1 g of the porous spherical silica was added and dispersed using an ultrasonic cleaner (BRANSONIC 1510J-DTH manufactured by BRANSON) for 30 minutes. The particle size distribution of this dispersed solution was measured using Multisizer III manufactured by Beckman Coulter. For the measurement, an aperture having an aperture diameter of 100 $\mu$m was used. From the obtained particle size distribution, the 50%, 10%, and 90% cumulative diameters of the volume-based particle size distribution were evaluated.

(Measuring Pore Volume and Pore Radius (Mode Pore Radius) by BJH, and BET Specific Surface Area)

The pore volume and the pore radius (mode pore radius) by BJH, and the BET specific surface area were measured with BELSORP-mini (manufactured by BEL JAPAN, Inc.) according to the aforementioned definitions.

(Compressive Test Forces when Specimens were Found to Break)

"Compressive test forces when specimens are found to break" were measured with a micro compression testing machine (MCT-W510-J manufactured by Shimadzu Corporation) according to the aforementioned definitions. The loading speed in the measurement was 0.4462 mN/sec, and the force-hold time was 10 seconds. For the measurement, an indenter of 200 $\mu$m in diameter was used.

(Alkali Metal Content)

To 1 g of the porous spherical silica, 10 mL of nitric acid and 10 mL of hydrofluoric acid were added to dissolve the silica. The resultant solution was heated at 180°C for 4 hours to evaporate to dryness. After the resultant was cooled to room temperature, 2 mL of nitric acid and 18 mL of ultrapure water were added, and then, 20 mL of a sample to be measured was obtained in a volumetric flask. The alkali metal content of the obtained sample was measured using an inductively coupled plasma-optical emission spectrometer (ICAP 650DUO manufactured by Thermo Scientific).

(Average Circularity)

A SEM image of at least 2000 particles of the porous spherical silica that was observed with a SEM (S-5500 manufactured by Hitachi High-Tech Corporation, 3.0 kV in acceleration voltage, secondary electron detection) at the magnification of 1000 was subjected to image analysis, and the average circularity was calculated according to the aforementioned definition.

(Number of Shred Particles (Number of Particles each Having Circularity of at most 0.7))

A SEM image of at least 2000 particles of the porous spherical silica that was observed with a SEM (S-5500 manufactured by Hitachi High-Tech Corporation, 3.0 kV in acceleration voltage, secondary electron detection) at the magnification of 800 was subjected to image analysis, and the circularity of each of the particles was calculated according to the aforementioned definition. The number of particles each having a circularity of at most 0.7 was counted.

<Example 1>

(Step of Preparing Dispersed Solution)

A fumed silica-dispersed solution was obtained by: while stirring, adding 66 g of REOLOSIL QS-30 (manufactured by Tokuyama Corporation) to 200 mL of an ion-exchanged water where 6.65 g of urea was dissolved using a homogenizer (T25BS1 manufactured by IKA) to predisperse a fumed silica; and thereafter, finely dispersing the resultant by the use of an ultrasonic homogenizer (Sonifier SFX250 manufactured by BRANSON). The particle size distribution of the solution after the dispersion was measured by a laser diffraction scattering method. As a result, the value of the D90 was 0.19 $\mu$m. This step of preparing a dispersed solution was performed in a chiller that was cooled to 10°C.

(Step of Preparing W/O Emulsion)

From the fumed silica-dispersed solution prepared by the foregoing method, 65.5 g of the solution was separated out. To the separated solution, 129 g of decane where 0.75 g of sorbitan monooleate (RHEODOL SP-O10V manufactured by Kao Corporation) was dispersed was added, and thereafter, stirred using a homogenizer at 8600 rpm for 3 minutes. A W/O emulsion was thereby obtained.

(Gelating Step)

The obtained W/O emulsion was kept in a water bath at 80°C for 3 hours while stirred at 200 rpm using four inclined-blades each having a blade diameter of 60 mm, a blade width of 20 mm, and an inclination of 45 degrees, thereby having gelated.

(Step of Collecting Gels)

To the resultant, 77 g of isopropyl alcohol and 52 g of water were added and stirred with stirring blades while kept at 70°C for 30 minutes. Thereafter, the resultant was allowed to stand, thereby separating into the two layers of an upper layer, which was an O-phase, and a lower layer, which was a W-phase.
Next, by decantation, the O-phase and the W-phase were separated, and the W-phase was collected.
The W-phase was subjected to suction filtration to sort gels out from the W-phase.

(Drying Step)

Cakes of the obtained gels were dispersed in 60 g of isopropyl alcohol, and put in a spray dryer (Mini Spray Dryer B-290 manufactured by BUCHI) while stirred with a stirrer. At this time, the concentration of the solid content of the silica after the drying in the slurry was 10 mass%. The inlet temperature of the spray dryer was set at 120°C, and the outlet temperature was 85°C. The feed rate of the slurry was 15 mL/min, and the circulating gas volume was 35 m$^3$/h.

(Firing Step)

The obtained dry powder was placed in a crucible, and the temperature thereof was raised at 5°C/min in heating rate with an electric furnace (FUS722PB manufactured by Advantech), and the dry powder was fired at 1000°C for 20 hours. The firing atmosphere was not adjusted but was the air atmosphere.

The physical properties of the porous spherical silica obtained in the foregoing way are shown in table 1 (the physical properties of the porous spherical silicas obtained in the following examples and comparative examples are also shown in table 1).

<Example 2>

A porous spherical silica was obtained in the same process as in example 1 except that the firing conditions were changed to 900°C for 17 hours. The outlet temperature in the spray-drying was 85°C.

<Example 3>

A porous spherical silica was obtained in the same process as in example 1 except that the rotation speed of the homogenizer in the step of preparing a W/O emulsion was changed to 3000 rpm. The outlet temperature in the spray-drying was 85°C.

<Example 4>

A porous spherical silica was obtained in the same process as in example 1 except that the rotation speed of the homogenizer in the step of preparing a W/O emulsion was changed to 10000 rpm. The outlet temperature in the spray-drying was 85°C.

<Example 5>

A porous spherical silica was obtained in the same process as in example 1 except that the firing conditions were changed to 1200°C for 15 hours. The outlet temperature in the spray-drying was 85°C.

<Example 6>

A porous spherical silica was obtained in the same process as in example 1 except that the rotation speed of the homogenizer in the step of preparing a W/O emulsion was changed to 6000 rpm, and the firing conditions were changed to 1200°C for 10 hours. The outlet temperature in the spray-drying was 85°C.

<Comparative Example 1>

By adding sulfuric acid that was 10 g/100 mL to a sodium silicate aqueous solution that was 9 g/100 mL and that had a molar ratio of $SiO_2/Na_2O$ of 3.1 so as to lead to a pH of 2.9, 500 mL of a silica sol was prepared. From this silica sol, 66.5 g of the silica sol was separated out. The step of preparing a W/O emulsion, the gelating step, and the step of collecting gels were carried out in the same manner as in example 1 except that the fumed silica-dispersed solution in example 1 was changed to that separated-out silica sol, and the resultant gels were obtained. The obtained gels were dried with a vacuum dryer, and fired at 1000°C for 20 hours, and the resultant porous spherical silica was obtained.

<Comparative Example 2>

A porous spherical silica was obtained by spray-drying a fumed silica-dispersed solution prepared in the same process as in the step of preparing a dispersed solution in example 1, and thereafter, firing the resultant at 1000°C for 20 hours.

<Comparative Example 3>

In example 1, the rotation speed of the homogenizer in the step of preparing a W/O emulsion was changed to 5500 rpm, and the gelating step and the step of collecting gels were carried out. Then, gels were obtained. The obtained gels were dried in a vacuum dryer, and fired at 1000°C for 20 hours. After fired, the resultant was disintegrated using a jet mill, and the resultant porous spherical silica was obtained.

<Comparative Example 4>

A porous spherical silica was obtained in the same process as in example 1 except that no firing step was carried out in example 1.

<Comparative Example 5>

A porous spherical silica was obtained in the same process as in example 6 except that the firing was performed at 800°C for 10 hours in example 6.

[Table 1]

(Table 1)

| | Particle diameter (D50) [μm] | D10/D90 | Pore volume [mL/g] | Mode pore radius [nm] | Specific surface area [m2/g] | Breaking compressive test force [mN] | Average circularity | Number of shred particles | Alkali metal content [ppm] |
|---|---|---|---|---|---|---|---|---|---|
| Example 1 | 10.2 | 0.60 | 0.7 | 14 | 125 | 14 | 0.87 | 5 | 30 |
| Example 2 | 9.8 | 0.45 | 1.8 | 17 | 230 | 11 | 0.90 | 8 | 22 |
| Example 3 | 25.3 | 0.46 | 1.5 | 15 | 132 | 13 | 0.83 | 3 | 12 |
| Example 4 | 5.6 | 0.38 | 1.2 | 15 | 153 | 13 | 0.88 | 9 | 23 |
| Example 5 | 10.5 | 0.44 | 1.1 | 15 | 195 | 15 | 0.85 | 6 | 17 |
| Example 6 | 12.3 | 0.41 | 0.9 | 14 | 173 | 17 | 0.83 | 8 | 20 |
| Comparative example 1 | 10.5 | 0.42 | 0.3 | 18 | 655 | 14 | 0.80 | 10 | 250 |
| Comparative example 2 | 9.5 | 0.25 | 1.8 | 22 | 257 | 0.5 | 0.85 | 9 | 10 |
| Comparative example 3 | 15.5 | 0.55 | 1.7 | 22 | 245 | 15 | 0.80 | 55 | 25 |
| Comparative example 4 | 10.2 | 0.35 | 1.8 | 22 | 255 | 0.42 | 0.88 | 9 | 35 |
| Comparative example 5 | 12.5 | 0.40 | 1.7 | 18 | 243 | 3.5 | 0.82 | 12 | 22 |

<Evaluation Results>

(Examples 1 to 6)

As shown in table 1, each of Examples 1 to 6 shows an increased hardness as the arithmetic mean value of "compressive test forces when specimens are found to break" was $1.0 \times 10^1$ to $2.0 \times 10^1$ mN where the specimens were ten particles, and the compressive test forces were obtained according to a method specified in JIS Z8844:2019 when the loading speed was set in 0.4462 mN/sec. Also in each of examples 1 to 6, the number of particles each having a circularity of at most 0.7 was at most 20 among the 200 particles observed in the SEM image; that is, the number of the shred particles was reduced. These were able to be realized by; drying gels obtained by gelating a fumed silica-dispersed solution in the solution by spray-drying under appropriate drying conditions as in the production method according to the present invention, thereby suppressing agglomeration of the particles; and carrying out firing under appropriate firing conditions.

Every porous spherical silica obtained in examples 1 to 6 was as follows: the D50 was in the range of 2 and 200 μm; the D10/D90 was at least 0.3; the pore volume by the BJH method was in the range of 0.5 and 8 mL/g; the mode pore radius by the BJH method was in the range of 5 nm and 50 nm; the specific surface area by the BET method was in the range of 50 m²/g and 400 m²/g; and the alkali metal content was at most 50 ppm.

In examples 1 to 6, the porous spherical silicas having various values of D50 were obtained. All the values of the D50 were controlled by adjusting the diameters of the droplets in the W/O emulsions by changing the rotation speeds in the emulsification. In the production method according to the present invention, the value of the D50 of the porous spherical silica can be easily controlled without any large change in production process.

(Comparative Example 1)

The porous spherical silica in comparative example 1, which used sodium silicate as a raw material, had a smaller pore volume. This was because the residue of sodium, which was derived from the raw material, caused melting point depression in the firing, and the pores were blocked. As described, when sodium silicate is used as a raw material, it is difficult to obtain a porous spherical silica having an increased hardness while maintaining a pore volume thereof.

(Comparative Example 2)

The porous spherical silica in comparative example 2, which was obtained by spray-drying the fumed silica-dispersed solution, had a low breaking compressive test force. This was because the strength of aggregation of primary particles of the base material before the firing was weak. As described, when the particles were made by spray-drying, it is difficult to obtain a porous spherical silica having the target hardness.

(Comparative Example 3)

The porous spherical silica in comparative example 3, which was obtained by being allowed to stand in a vacuum dryer to be dried after the gels were collected, the number of shred particles was increased. This was because the particles agglomerated in the stationary drying, and performing the disintegrating step leads to generation of shred particles. As described, it is difficult to obtain a porous spherical silica including a smaller number of shred particles in stationary drying.

(Comparative Example 4)

The porous spherical silica in comparative example 4, for which no firing step had been carried out, had a low breaking compressive test force. As described, it is difficult to obtain a porous spherical silica having an increased hardness unless the firing step is carried out.

(Comparative Example 5)

The porous spherical silica in comparative example 5 had a low breaking compressive test force. As described, it is difficult to obtain a porous spherical silica having an increased hardness unless the firing conditions are proper.

**Claims**

1. A porous spherical silica, wherein

a 50% cumulative diameter (D50) of volume based particle size distribution measured by a Coulter counter method is in a range of 2 $\mu$m and 200 $\mu$m,

a ratio (D10/D90) of a 10% cumulative diameter (D10) of the distribution to a 90% cumulative diameter (D90) of the distribution is at least 0.3,

a pore volume by a BJH method is 0.5 mL/g to 8 mL/g,

a mode pore radius by the BJH method is 5 nm to 50 nm,

a specific surface area by a BET method is 50 $m^2$/g to 400 $m^2$/g, and

an arithmetic mean value of "compressive test forces when specimens are found to break" is $1.0 \times 10^1$ mN to $2.0 \times 10^1$ mN, the specimens being ten particles, the compressive test forces being obtained according to a method specified in JIS Z8844 when a loading speed is 0.4462 mN/sec,

a number of particles each having a circularity of at most 0.7 is at most 20, the particles being among 200 particles observed in an SEM image, and

an alkali metal content is at most 50 ppm.

2. A catalyst carrier comprising the porous spherical silica according to claim 1.

3. A cosmetic comprising the porous spherical silica according to claim 1.

4. An analytical column comprising the porous spherical silica according to claim 1.

5. A polish comprising the porous spherical silica according to claim 1.

6. A resin composition comprising the porous spherical silica according to claim 1.

7. A method of producing a porous spherical silica, the method comprising:

preparing a W/O emulsion comprising an aqueous phase where a fumed silica is dispersed, and an organic phase including a nonaqueous solvent as a major component;

heating the emulsion and gelating the aqueous phase to obtain a gel-dispersed solution;

collecting, from the solution, and spray-drying generated gels; and

firing obtained porous spherical silica at a temperature of 900 to 1500°C for 10-25 hours.

8. The method of producing a porous spherical silica according to claim 7, wherein
a spraying method in the spray-drying is by a method using a two-fluid nozzle.

## INTERNATIONAL SEARCH REPORT

| | International application No. |
| --- | --- |
| | **PCT/JP2024/007618** |

**A. CLASSIFICATION OF SUBJECT MATTER**

*C01B 33/18*(2006.01)i; *A61Q 1/00*(2006.01)i; *A61Q 5/00*(2006.01)i; *A61Q 11/00*(2006.01)i; *A61Q 19/00*(2006.01)i; *B01J 20/283*(2006.01)i; *B01J 32/00*(2006.01)i; *B01J 35/60*(2024.01)i; *B01J 37/00*(2006.01)i; *B01J 37/08*(2006.01)i
FI:  C01B33/18 E; B01J37/00 F; B01J35/60 Z; B01J37/08; B01J32/00; A61Q1/00; A61Q19/00; A61Q5/00; A61Q11/00; B01J20/283

According to International Patent Classification (IPC) or to both national classification and IPC

**B. FIELDS SEARCHED**

Minimum documentation searched (classification system followed by classification symbols)

C01B33/00-33/193; A61Q1/00-90/00; A61K8/00-8/99; B01J20/283; B01J21/00-38/74

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2022/154014 A1 (TOKUYAMA CORPORATION) 21 July 2022 (2022-07-21)<br>entire text | 1-8 |
| A | WO 2019/131873 A1 (JGC CATALYSTS & CHEMICALS LTD.) 04 July 2019 (2019-07-04)<br>entire text | 1-8 |
| A | JP 2020-193144 A (NOURYON CHEMICALS INTERNATIONAL BV) 03 December 2020 (2020-12-03)<br>entire text | 1-8 |
| A | JP 2014-210671 A (TOKUYAMA CORPORATION) 13 November 2014 (2014-11-13)<br>entire text | 1-8 |
| A | JP 2010-100503 A (COVALENT MATERIALS CORP.) 06 May 2010 (2010-05-06)<br>entire text | 1-8 |

☑ Further documents are listed in the continuation of Box C.      ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **22 April 2024** | **14 May 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2024/007618** |

### C. DOCUMENTS CONSIDERED TO BE RELEVANT

| Category* | Citation of document, with indication, where appropriate, of the relevant passages | Relevant to claim No. |
| --- | --- | --- |
| A | JP 2020-79165 A (KAO CORPORATION) 28 May 2020 (2020-05-28)<br>entire text | 1-8 |
| A | WO 2009/072218 A1 (JGC CATALYSTS & CHEMICALS LTD.) 11 June 2009 (2009-06-11)<br>entire text | 1-8 |

Form PCT/ISA/210 (second sheet) (July 2022)

## INTERNATIONAL SEARCH REPORT
### Information on patent family members

International application No.

**PCT/JP2024/007618**

| Patent document cited in search report | | | Publication date (day/month/year) | Patent family member(s) | | | Publication date (day/month/year) |
|---|---|---|---|---|---|---|---|
| WO | 2022/154014 | A1 | 21 July 2022 | US | 004279455 | A | |
| | | | | entire text | | | |
| | | | | CN | 116406344 | A | |
| | | | | KR | 10-2023-0128444 | A | |
| WO | 2019/131873 | A1 | 04 July 2019 | US | 2021-0094835 | A1 | |
| | | | | entire text | | | |
| | | | | CN | 111527047 | A | |
| | | | | KR | 10-2020-0102445 | A | |
| JP | 2020-193144 | A | 03 December 2020 | US | 2020/0376464 | A1 | |
| | | | | entire text | | | |
| | | | | EP | 003744684 | A1 | |
| | | | | entire text | | | |
| | | | | CN | 112010315 | A | |
| | | | | KR | 10-2020-0138662 | A | |
| JP | 2014-210671 | A | 13 November 2014 | (Family: none) | | | |
| JP | 2010-100503 | A | 06 May 2010 | (Family: none) | | | |
| JP | 2020-79165 | A | 28 May 2020 | (Family: none) | | | |
| WO | 2009/072218 | A1 | 11 June 2009 | US | 2010-0247914 | A1 | |
| | | | | entire text | | | |
| | | | | EP | 002228344 | A1 | |
| | | | | entire text | | | |
| | | | | KR | 2010-0106335 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004101139 A1
- WO 2019131873 A1
- WO 2022154014 A1

**Non-patent literature cited in the description**

- *Journal of the Society of Powder Technology.*, 1988, vol. 25 (12), 805-811
- **BARRETT, E. P.** ; **JOYNER, L. G.** ; **HALENDA, P. P.** *J. Am. Chem. Soc.*, 1951, vol. 73, 373